(19) Europäisches Patentamt / European Patent Office / Office européen des brevets

(11) **EP 3 906 853 A1**

(12) **EUROPEAN PATENT APPLICATION**

(43) Date of publication:
10.11.2021 Bulletin 2021/45

(51) Int Cl.:
*A61B 5/1455* (2006.01)    *A61B 5/11* (2006.01)
*A61B 5/00* (2006.01)

(21) Application number: 20305439.0

(22) Date of filing: 05.05.2020

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
KH MA MD TN

(71) Applicant: **Withings**
**92130 Issy-les-Moulineaux (FR)**

(72) Inventors:
• EDOUARD, Paul
92130 ISSY LES MOULINEAUX (FR)
• PEREZ, Gaël
92130 ISSY LES MOULINEAUX (FR)
• LELONG, Martin
92130 ISSY LES MOULINEAUX (FR)

(74) Representative: **Plasseraud IP**
**66, rue de la Chaussée d'Antin**
**75440 Paris Cedex 09 (FR)**

(54) **METHOD AND DEVICE TO DETERMINE SLEEP APNEA OF A USER**

(57) A method to determine breath disturbances of a user, in a device comprising three LEDs (11,12,13) configured to emit light rays at three wavelengths, a photodiode (2,3), a control unit (6) and a motion sensor (14,15), the method comprising :

a- acquiring, at a predefined sampling frequency, a PPG light response for each wavelength, resulting in a first, second and third raw data series representing an amplitude response, and acquiring motion signals, resulting in a fourth raw data series,

b- applying feature extracting filters to the raw data series,

for outputting pattern channels comprising:
- variation (DSpO2) of blood oxygen saturation level,
- variation (DRSA) of Respiratory Sinus Arrythmia,
- variation (DRR) of Respiration Rate,
- Motion index,
- Heart Rate Variation,
- variation of AC and/or DC component of PPG light response,

c- applying a 1D CNN to the pattern channels for determining a breathing disturbance event and AHI.

FIG. 5

## Description

### Technical Field

This disclosure pertains to the field of devices allowing to detect and identify breathing disturbances of a user, notably apnea and/or hypopnea events of an individual at sleep. More precisely, this disclosure is directed to a single/integral device non invasively worn by a user and capable of detecting and identifying breathing disturbances of such user.

### Background Art

Sleep apnea is a sleep disorder where a person has pauses in breathing (apnea event) or periods of shallow breathing (hypopnea event) during sleep. There are three forms of sleep apnea: obstructive (OSA) central (CSA), and a combination of the two called mixed. In OSA, breathing is interrupted by a blockage of airflow, while in CSA breathing stops from a lack of effort to breathe.

The prevalence of Sleep Apnea is estimated to be in the range of 3% to 7% in adults with certain subgroups of the population bearing higher risk. The screening of this disorder is most often made using polysomnography or polygraphy, both methods requiring to wear impractical sensors. Because of this, Sleep Apnea Syndrome (SAS) is an underdiagnosed disease.

In US2019282159 it is proposed to detect breathing disturbances. However, it relies on Fourier transform and/or so-called multi spectra tracker, which involves powerful calculation resources.

The inventors have sought to propose methods and devices suitable for accurate breathing disturbances determination, at various measuring anatomical sites, like for example and non exclusively : wrist, forehead, earlobe, ear-canal, ankle, and with optimized resources allocated, i.e. with a limited amount of computation resources and memory resource.

### Summary of the disclosure

To this aim, it is promoted herein a method to detect breathing disturbances of a user, carried out in a single/integral device comprising a plurality of LEDs (11,12,13) configured to emit light rays at least three wavelengths ($\lambda$1, $\lambda$2, $\lambda$3), at least a light sensing device (e.g. photodiode), a control unit and at least a motion sensor (14,15), the method comprising :

a- acquiring, at a predefined sampling frequency, a photoplethysmography (PPG) light response for each wavelength of the plurality of at least three wavelengths, resulting respectively in a first, second and third raw data series representing an amplitude response through a user's tissue, and acquiring motion signals from the motion sensor, resulting in a fourth raw data series,

b- applying, at the control unit (6), a plurality of feature extracting filters to the first, second, third and fourth raw data series, each of the plurality of feature extracting filters outputting one of a plurality of corresponding pattern channels, the plurality of pattern channels comprising at least one or more of :

- variation (DSpO2) of blood oxygen saturation level (SpO2),
- variation (DRSA) of Respiratory Sinus Arrythmia (RSA),
- variation (DRR) of Respiration Rate (RR),
- Heart Rate Variation (HRV),
- Motion index, / Motion composite index
- variation of AC component of PPG light response,
- variation of DC component of PPG light response,

c- applying, at the control unit (6), a computation engine (5) to the plurality of pattern channels, and detecting therefrom a breathing disturbance event, wherein the computation engine comprises a neural network.

Thanks to this arrangement, the use of digital feature extracting filters together with a fairly simple computation engine allows a reliable and accurate assessment of a user breathing disturbance event. The above digital filters and the neural network are used in a subsequent manner. The use of three wavelengths PPG response allows a reliable and accurate collection of data useful for the assessment of a user breathing disturbance event. The device performing the promoted method can be rather small and adapted to be worn in a user-friendly manner to accompany the user when he/she sleeps. The device performing the promoted method is cost effective since it requires only a low computation and memory resources.

The light intensity received at the light sensing device and the motion signals are converted into a digital value by a ADC (Analog to Digital Converter), so resulting data can be readily handled by digital computation means.

The following features, can be optionally implemented, separately or in combination one with the others.

According to one option, the neural network of the computation engine (5) comprises a one dimension convolutional Neural Network (1DCNN). We use an optimized Neural Network requiring a limited amount of resources, in terms of memory and computing power.

According to another option, the neural network can comprise a LSTM neural network, i.e. a Long Short Term Memory neural network. We may use recurrence provided in LSTM neural network to get another optimal solution for the computation engine.

According to one option, the method may further comprise a step of determining an Apnea/Hypopnea Index (AHI) computed from a calculation of a number of breathing disturbance event(s) (BDE) over a total/cumulated sleep duration. A synthetic report of the past night can therefore be issued for the user and/of for its caretaker. The synthetic report of the past night is considered a score of sleep quality and/or reveal light or severe apnea syndrome.

According to one option, the plurality of pattern channels comprises at least:

- variation (DSpO2) of blood oxygen saturation level (SpO2),
- variation (DACPPGi) of AC component of PPG light response, said PPG light response being the green wavelength PPG light response,
- variation (DDCPPGi) of DC component of PPG light response, said PPG light response being the green wavelength PPG light response,

and the plurality of pattern channels comprises at least one or more of:

- variation (DRSA) of Respiratory Sinus Arrythmia (RSA),
- variation (DRR) of Respiration Rate (RR),
- Heart Rate Variation (HRV),
- Motion index.

Green PPG light response and its variations can be used as a key data for assessing a breathing disturbance event.

According to one option, the one dimension convolutional Neural Network (1DCNN) comprises an input layer fed by latest values of the plurality of pattern channels. Thereby we use a CNN with a limited and reasonable number of dimensions and it can be embedded in a cost effective computation unit.

According to one option, the one dimension convolutional Neural Network exhibits less than 5000 weight coefficients, and exhibits less than 2500 floating point multiplications per second. Such CNN requiring a limited amount of resources, in terms of memory and computing power can be embedded in a low cost computation unit.

According to one option, there is provided a deep learning process, configured to tweak the various weights involved in the one dimension convolutional Neural Network (1DCNN), is carried out from a set patients undergoing polysomnography with various additional sensors (clinical tests).

According to one option, the method may further comprise one or more of:

- displaying the Apnea/Hypopnea Index (AHI) on a display intended to be looked at by a user
- providing Apnea/Hypopnea Index (AHI) to a remote entity, preferably via a wireless transmission.

Results are therefore displayed directly to the user/wearer and can be forwarded to another device either belonging to the user or to some medical staff/caretaker.

According to one option, the feature extracting filters run on stream on signal circular buffer values, each comprising one or more of : exponential filtering, maxima detection, normalization, nonlinear rescaling. Such type of feature extracting filters are lightweight algorithms.

According to one option, the promoted method is carried out regardless of any user profile. The inventors have found that the promoted method works well irrespective of a user profile (gender, weight, height, BMI, etc).

According to one option, the pattern channels, outputted by the feature extracting filters, comprises a downsampling from the first sampling frequency (F1) to a second frequency (F2), said second frequency being comprised between 0,5 Hz and 2Hz, preferably around 1 Hz. Since the sampling time can be rather long (several hours of sleep), we combine a rather high primary sampling rate for good data collection with a rather low computation frequency to save electrical energy.

According to one option, the plurality of at least three wavelengths ($\lambda 1$, $\lambda 2$, $\lambda 3$) comprises :

- a first wavelength ($\lambda 1$) which has a center emission wavelength comprised between at 920 nm and 960 nm and forms an infrared LED,
- a second wavelength (λ2) which has a center emission wavelength comprised between at 650 nm and 665 nm and forms a red LED,
- a third wavelength (λ3) which has a center emission wavelength comprised between 480 nm and 540 nm and forms a green or blue LED.

We therefore use substantial differences regarding hemoglobin absorption index between infrared and red wavelengths to output SPO2 and variations thereof, and besides we use the strong pulsatile signal given by the green wavelength.

According to one option, the light response for each wavelength are taken at a user's wrist. This turns to be a practical location for permanent wear, and also a user-friendly location.

According to one option, the light response for each wavelength are taken at a user's forehead. A head band with forehead applied sensor(s) turns to be a practical location for permanent wear for the night.

According to one option, steps a- to c- are implemented in a control unit housed in a wrist-worn device. A stand-alone unit can perform all the process of the promoted method.

According to one option, the wrist-worn device is a wristwatch. Further, the device can give current time; further such the device is good looking and does not look like a medical device although it can give a highly valuable apnea feedback.

According to one option, the method may further comprise a worn test function, wherein whenever the worn test function gives a negative result, at least displaying and outputting of the user breathing disturbance event and/or Apnea/Hypopnea Index (AHI) are suspended, and possibly the computation is also suspended. It is useless to display a result with no significance whenever the device is not worn on user's tissue. Further, electrical energy can be spared whenever the device is not worn.

According to one option, wherein the predefined first sampling frequency (SF) is comprised between 15 Hz and 200Hz, preferably comprised between 15 Hz and 50Hz, more preferably comprised between 20 Hz and 30Hz. Accordingly, we have a good compromise regarding accuracy and electrical energy consumption.

According to one option, wherein at the step a-, the acquisition of PPG photoplethysmography light response is performed by two photodiodes, i.e. a broadband photodiode (2) configured to receive red and infrared light rays, and a selective photodiode (3) configured to receive green light rays. This leads to optimization of photodiode usage, selective photodiode is dedicated to green wavelength, sensitivity of broadband photodiode is interesting for red and infrared wavelengths.

According to one option, at least step **b-** is performed each time a new set of wavelength light responses is acquired and motion signals are acquired, namely feature extracting filters are timely triggered according to the first sampling frequency clock.
Simple task handling within the control unit. Optimal electrical energy consumption.

According to another aspect, the disclosure is also directed at a device comprising a plurality of LEDs (11,12,13) configured to emit light rays at least three wavelengths (λ1, λ2, λ3), at least a light sensing device (/photodiode)(2,3), a control unit (6), and at least a motion sensor (14,15), the device being configured to carry out a method as defined above.

**Brief Description of Drawings**

Other features, details and advantages will be shown in the following detailed description and on the figures, on which:

[Figure 1] is a side elevation view of an apnea sensing wristwatch according to the present disclosure.

[Figure 2] illustrates such a watch worn by a user at the wrist and having a communication interface.

[Figure 3] shows more in details a back view of the watch centered on the glass window.

[Figure 4] shows more in details an elevation sectional of the wristwatch back case bearing on the wrist skin.

[Figure 5] shows a functional block diagram.

[Figure 6] shows a bottom perspective view of the apnea sensing wristwatch of figure 1.

[Figure 7] shows an absorption chart relating to Hemoglobin.

[Figure 8] shows one of the wavelength data series, raw, DC and AC.

[Figure 9] shows a functional block diagram partly focused on PPG signals decomposition.

[Figure 10] shows a functional illustration of a one dimension convolutional Neural Network

[Figure 11] shows a functional block diagram of the oximeter wristwatch of figure 1.

[Figure 12] shows a time chart illustrating the motion signals and the selective activation of the various wavelengths LEDs.

[Figure 13] shows a functional block diagram partly focused on AHI calculation.

[Figure 14] shows a time chart of a PPG signal together with some breath disturbances.

[Figure 15] shows a time chart of night synthesis about apnea.

[Figure 16] shows a time chart with more than one respiration cycle together with corresponding heart cycles, illustrating the Respiratory Sinus Arrythmia.

**Description of Embodiments**

Overall and PPG basics

As shown on some figures, an apnea sensing wristwatch **9** is provided. As will be exposed below, the wristwatch exhibits an apnea sensing function, namely an assessment of a breathing disturbances of a user.

The user can be here a patient under medical surveillance or a user interested in having a better knowledge of his/her sleep quality.

We note also that the wristwatch can also possibly exhibit an SPO2 function (real time assessment of the user's functional blood oxygen saturation level) and possibly an ECG function (i.e. Electro Cardio Gram).

However, the wristwatch is only one example among others, regarding devices for measuring one or more bio-parameter(s) including assessing a breathing disturbance.

Other anatomical site(s) different from the wrist is also considered. Other types of device(s) besides wristwatch or wristband are also considered. A nonlimiting list can be : forehead, earlobe, ear-canal, ankle, etc..

The device can be a head band with forehead-applied sensor(s). The device can be a hearing head or ear buds and the like. The device can be an armcuff.

The measuring device can applied momentarily, on the long run or permanently to the anatomical site(s).

We use a technique termed photoplethysmography or, in short, "PPG" for obtaining the above one or more physiological parameters reflecting blood circulation in the tissue of the anatomical site(s)).

PPG involves a non-invasive optical sensor that detects the light response from within a patient's tissue indicative of the amount of light absorbed within the tissue at the illuminated site.

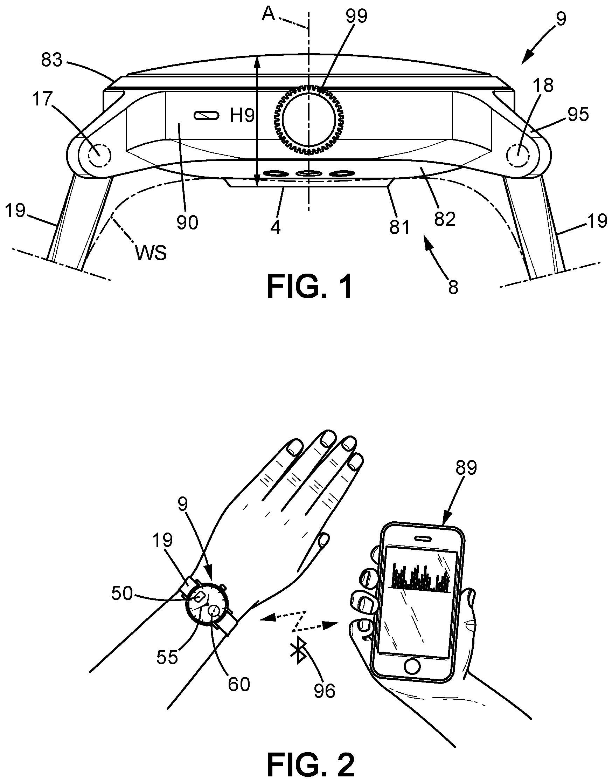

Turning back to the wristwatch illustrated example (Figs 1-4 and 6), there is provided a main casing **90** and a wristband **19** attached thereto. Further there is provided, attached to the main casing **90** of the wristwatch, a back-case assembly generally denoted **8**. The back-case assembly **8** comprises, from the center, going radially outwards: a lens **4**, a ring member **81** and an annular cup member **82.**

The lens **4** forms a transparent or translucent window allowing optical interaction between the wristwatch and the environment at its back; notably the wrist skin **WS.**

Behind the lens **4**, there is provided a light emission assembly **1**. The light emission assembly 1 comprises a green LED **13**, a red LED **12** and an infrared LED **11.** LED stands for Light Emission Diode.

As will now be seen below, we use several wavelengths.

In the present disclosure, the infrared LED **11** has a center emission wavelength comprised between 920 nm and 960 nm. According to one particular example, the infrared LED 11 has a center emission wavelength at 940 nm.

More generally speaking, we use a first wavelength denoted $\lambda 1$, in the infrared wavelength part of the spectrum.

In the present disclosure, the red LED **12** has a center emission wavelength comprised between 650 nm and 665 nm. According to one particular example, the red LED 12 has a center emission wavelength at 655 nm.

More generally speaking, we use a second wavelength denoted $\lambda 2$, in the red wavelength spectrum.

In the present disclosure, the green LED **13** has a center emission wavelength comprised between 480 nm and 540 nm. According to one particular example, the green LED 13 has a center emission wavelength at 530 nm.

It is to be understood in the present disclosure that "green" has been given a broad scope, also encompassing partly of the blue wavelengths, at least the blue/green nuances.

More generally speaking, we use a third wavelength denoted λ**3**, in the green/blue wavelength spectrum.

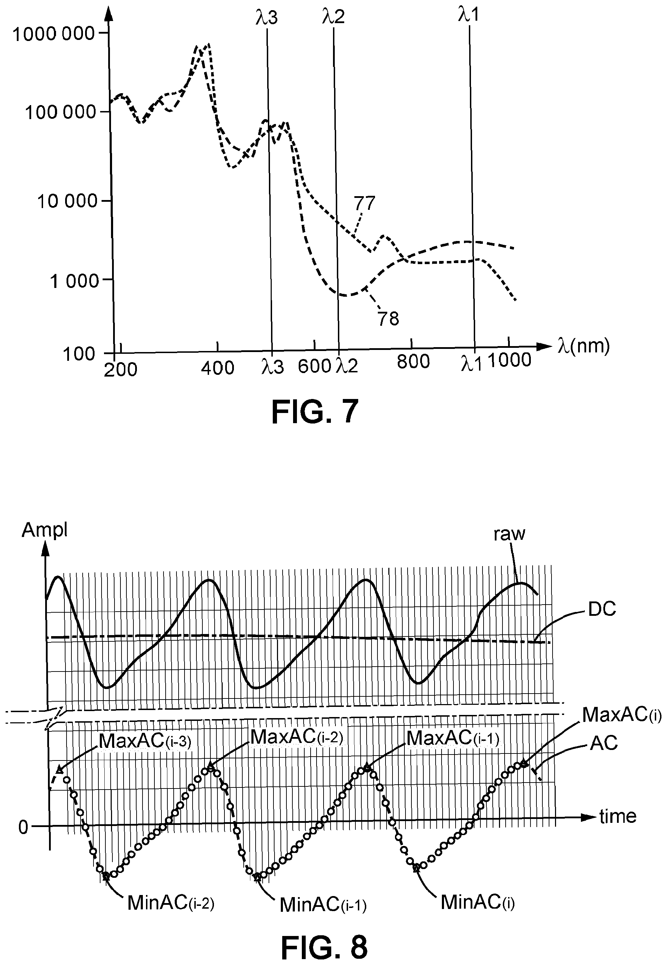

As apparent from Figure 7, we note that the three wavelengths λ**1**, λ**2**, λ**3** are spread/distributed across the light spectrum (visible + infrared).

On figure 7, curve **77** denotes the absorption index of deoxygenated Hemoglobin, whereas curve **78** denotes the absorption index of oxygenated Hemoglobin. The difference between the 2 curves influences the PPG responses.

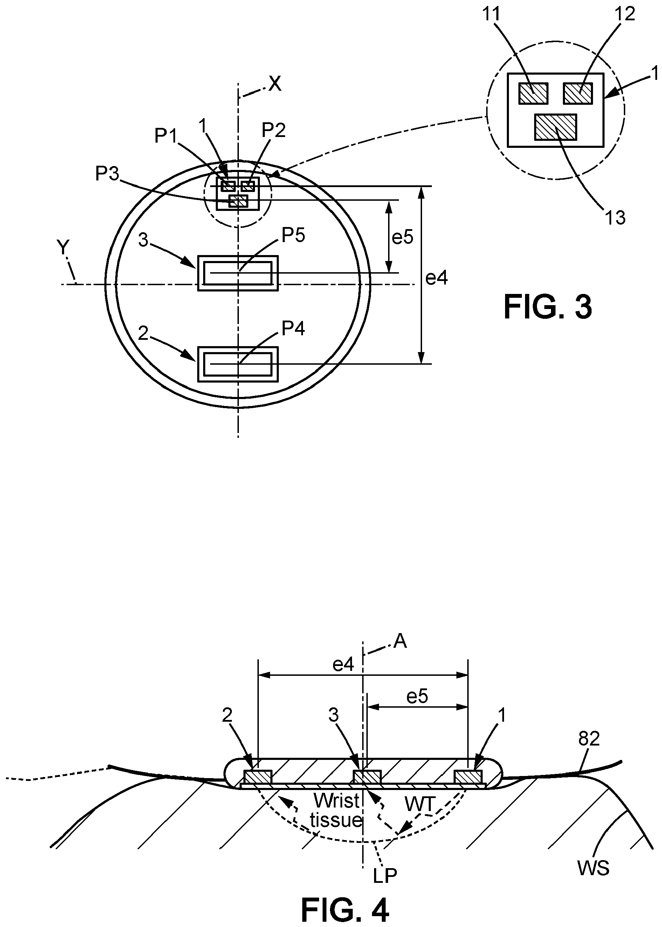

Each LED is configured to emit light rays toward wrist tissue **WT** through the lens 4, and some light is perfused through the site tissue. There is provided at least a broadband photodiode **2** configured to receive light rays. From the LED, there is defined a Light Path denoted **LP** from the LED to the broadband photodiode (cf. Fig 4). Only one photodiode, i.e. a single photodiode, can be used to receive the three wavelengths light rays.

In the shown example, the acquisition of PPG photoplethysmography light response is performed by two photodiodes, i.e. a broadband photodiode 2 configured to receive red and infrared light rays, and a selective photodiode 3 configured to receive green light rays.

For an optimization of photodiodes usage, selective photodiode 3 is dedicated to green wavelength, sensitivity of broadband photodiode **2** is interesting for red and infrared wavelengths.

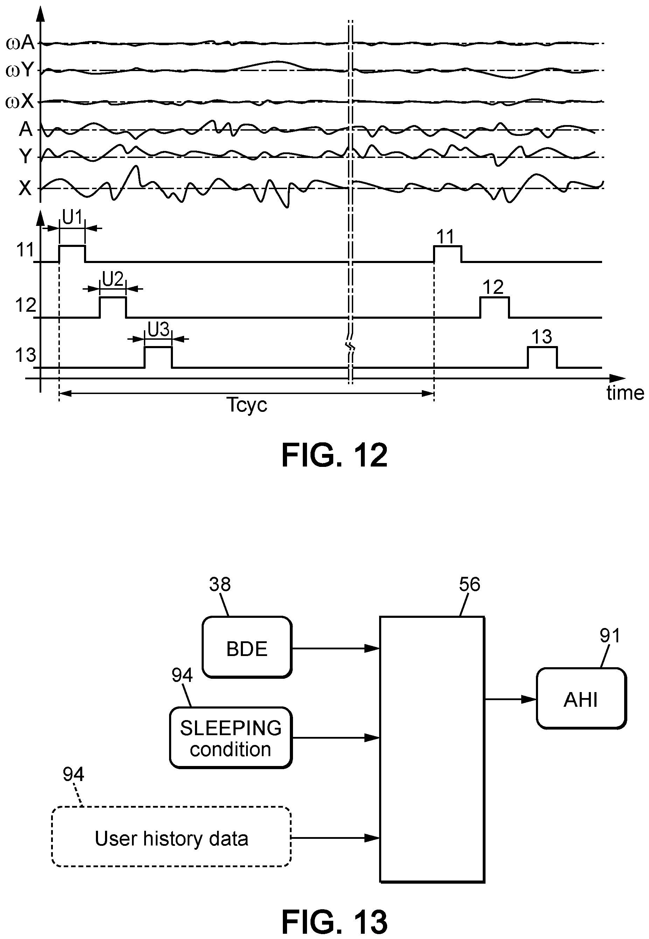

As shown at Figure 12, each **LED** is selectively activated, one after another. Wavelengths are timely segregated and optical crosstalk is reduced. For one measure, each LED is activated for a short time, i.e. a pulse. According to the illustrated example, first the infrared LED is activated for a duration **U1**, comprised between 50 microseconds and 200 microseconds. Then the red LED is activated for a duration **U2**, comprised between 50 microseconds and 200 microseconds. Then the green LED is activated for a duration **U3**, comprised between 50 microseconds and 200 microseconds. LED activation phases are separated by a pause time that can be for example comprised between 10 microseconds and 50 microseconds.

After the three LED activation pulses, calculation steps take place as will be discussed later.

Sampling overall process is repeated with a sequence duration **Tcyc**. **Tcyc** corresponds to sampling frequency **F1** (i.e. 1/Tcyc). The sampling frequency **F1**, named here 'first' sampling frequency is comprised between 15 Hz and 200 Hz, preferably comprised between 15 Hz and 50 Hz, more preferably comprised between 20 Hz and 30 Hz.

In the illustrated example, the first sampling frequency **F1** is 25 Hz, which is a good compromise regarding accuracy and electrical energy consumption. **Tcyc** is 40 milliseconds in this example.

Higher sampling frequencies are also possible, namely above 40 Hz up to hundreds of Hz.

It is to be noted that the current flowing in each **LED** when each **LED** can be interestingly selectively adaptively controlled. The PPG function can be readily adapted to the skin nature and color/tone of a large scope of users.

Motion sensor and other sensing means

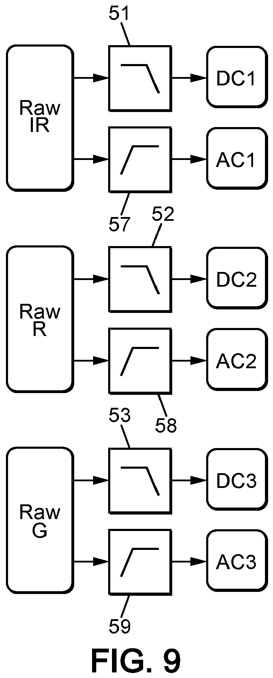

The motion sensor, also called generically "accelerometer" is represented at Figure 9 as part of the control unit 6. In the illustrated example, the motion sensor is a 6 axis sensor. A first part denoted **14** is able to measure linear accelerations along the three perpendicular directions **X, Y, A**. A second part denoted **15** is able to measure the rotation motion around the three perpendicular axis **X, Y, A,** denoted respectively ω**X, wY** ω**A**.

Miniature gyrometers are used for rotation motions ω**X, wY** ω**A**.

In one example, first part **14** and second part **15** are integrated in a single electronic package. In one example, the motion sensor only includes the first part **14**. In another example, the motion sensor only includes the second part **15**.

In one example, said single electronic package is a SMD miniature device soldered on the main PCB of the device.

The motion signals sensed at the linear accelerometers and gyrometers are converted into a digital value by a ADC **16** (Analog to Digital Converter), so it can be readily handled by digital computation means.

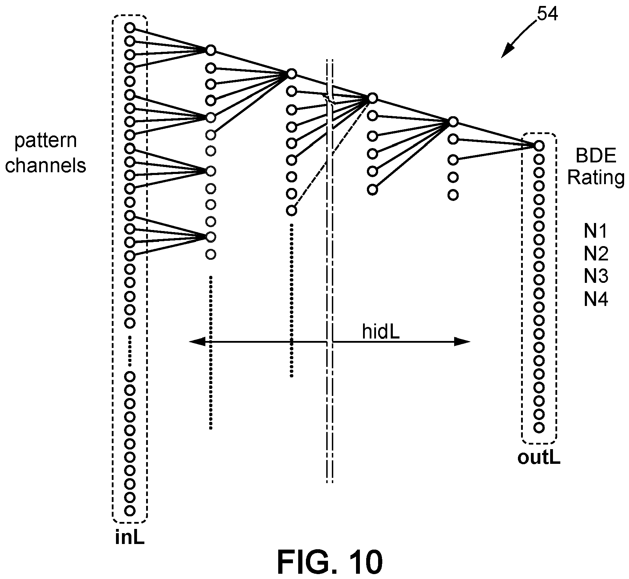

Some examples of raw signals are shown at the top lines at Figure 10.

Sampling process of motion signal is repeated with a sequence duration **Tcyc**. **Tcyc** corresponds to the first sampling frequency **F1** (i.e. 1/Tcyc), preferably the same as per the PPG signals sampling.

In a configuration of a wrist worn device, motion signals are representative of the movements of the forearm and the wrist.

In a configuration of a device placed at the forehead, for example a sleep monitoring head band, motion signals are representative of the movements of the head of the user/patient.

In a configuration of a device placed at the ear, for example a hearing aid or ear buds, motion signals are representative of the movements of the head of the user/patient.

There may be provided additionally a temperature sensor denoted **97**. The temperature sensor senses the temperature of the user's skin **WS**.

There may be provided additionally an acoustic sensor denoted **84**. The acoustic sensor, formed for example as a microphone, senses noises in the environment, including snoring noises made by the user/patient.

Motion signals, together optionally with the temperature signal are used to determine if the user/patient is sleeping. More details about this determination can found in document US2015289802 of same applicant which deals about wrist worn determination and sleep status sensing and determination.

In the present disclosure and in the frame of the promoted method, we don't need to determine particularly the sleep phases (REM, Light sleep, deep sleep); the apnea or hypopnea event can occur equally at any phase. However, the method operates as soon as the user falls or is asleep.

There are provided three electrodes for an ECG function : the ring member **81,** the annular cup member **82** and a front bezel ring **83.** Although ECG function is not used for detecting breath disturbance, ECG function is provided in a same single device.

Control unit, sampling and overall computation

Further, there is provided a control unit **6,** housed in a wristwatch or in any other device like a hearing aid, a neck lace, a sleep monitoring head band, an activity tracker. The control unit **6** is configured to control the LEDs, receive response signal at photodiode(s), receive motion signal from motion sensor and perform various computation steps.

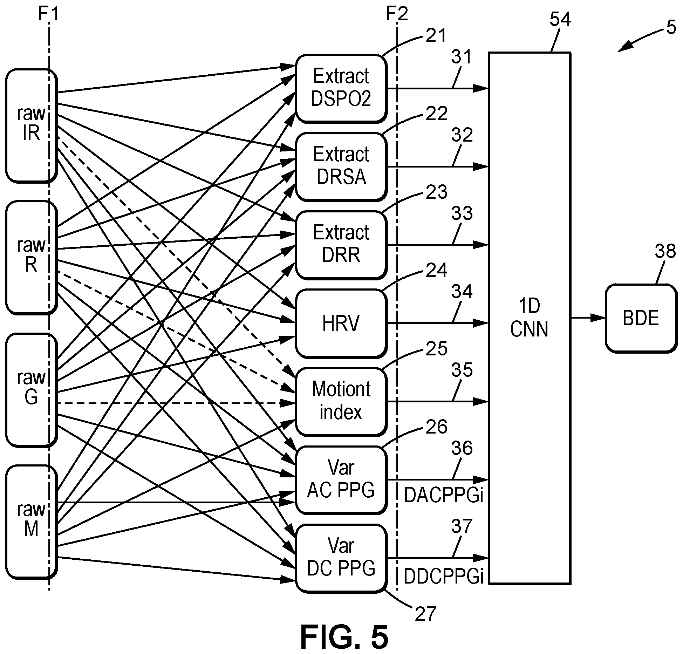

The overall computation process is illustrated at figures 5 and 13.

More precisely, the control unit **6** is configured to collect electrical signals delivered by the selective and broadband photodiodes **(2,3)** upon selective activation of each LED of the light emission assembly **1.**

As time passes, the sampling process collects periodically three light response values motion signal values. Basic sampling at first sampling frequency **F1** is named step a- of the promoted method.

The light intensity received at the photodiode is converted into a digital value by a ADC **16** (Analog-to-Digital Converter), so it can be readily handled by computation means. Also the motion signals sensed at the linear accelerometers and gyrometers are converted into a digital value by an ADC (Analog-to-Digital Converter), so it can be readily handled by digital computation means.

The collected data is named raw data, as shown at figure 5; the raw data series are respectively denoted **rawG** for the green wavelength, **rawR** for the red wavelength **rawIR** for the infrared wavelength. Further motion raw signals are denoted **rawM** at figure 5.

For the three first series (PPG) the raw data series is to be decomposed in a DC data series and AC data series. DC stands for 'continuous' or 'unmodulated' component, whereas AC stands for 'alternative' or 'unmodulated' or 'pulsatile' component.

For the fourth raw data source, the motion raw signals **rawM** is to be decomposed in a DC data series and AC data series.

Feature extracting filters

As promoted herein, and illustrated at Fig 5, the control unit 6 uses one or more feature extracting filters that take as input one or more of the first to fourth raw data series, contained in a circular buffer of the latest sampled values, representing notably a time window. This time window can be comprised between 2 seconds and 20 seconds.

More precisely, we define a step **b-** of the method in which we apply a plurality of feature extracting filters (refs **21** to **27**) to the first, second, third and fourth raw data series, each of the plurality of feature extracting filters outputting one of a plurality of corresponding pattern channels (refs **31** to **37**).

The plurality of outputted pattern channels comprise at least one or more of the following:

**31-** Variation (DSpO2) of blood oxygen saturation level (SpO2),
**32-** Variation (DRSA) of Respiratory Sinus Arrythmia (RSA),
**33-** Variation (DRR) of Respiration Rate (RR),
**34-** Heart Rate Variation (HRV),
**35-** Motion index,
**36-** variation (DACPPGi) of AC component of PPG light response,
**37-** variation (DDCPPGi) of DC component of PPG light response.

More precisely, a first feature extracting filter **21** computes a variation (DSpO2) of blood oxygen saturation level **31**. This calculation can be based notably on AC and DC components of the infrared and red light PPG responses.

Variation (DSpO2) of blood oxygen saturation level can be at first order Diff (R) = [AC2 / DC2] / [AC1 / DC1], ACi and DCi components are obtained as explained below.

As apparent from Figure 9, there are provided a first low-pass filter **51** which gives as output a first DC data series **DC1,** a second low-pass filter **52** which gives as output a second DC data series **DC2** a third low-pass filter **53** which gives as output a third DC data series **DC3.**

On the other hand, the AC component is also to be extracted from each raw data series.

According to one possibility, a high-pass filter is applied directly to the raw data series each to give a resulting AC data series representing a modulated amplitude response, namely a first AC data series **AC1,** a second AC data series **AC2** and a third AC data series **AC3.** High-pass filter are respectively denoted **57, 58, 59**

Alternately, for each wavelength series, the DC data series is subtracted from the raw data series to give a resulting AC data series representing a modulated amplitude response.

Figure 8 gives an example of a light PPG signal, where the raw signal 'Raw' sampled at 25 Hz is decomposed in a **DC** data series and a **AC** data series. This is done for the three PPG light responses. This can also be done for motion signal to discard the gravity component.

Turning back to the first pattern channel, it has been found that SpO2 goes down during respiratory events. Hence a significant decrease of SPO2, i.e. DSPO2 significantly negative, denotes an apnea event just occurred and/or still ongoing.

At second order, Variation (DSpO2) of blood oxygen saturation level can be influenced by motion of the user, significance is reduced when motion index is above a certain threshold. Also green light response can be used to synchronize red and IR signals at low level.

Further, a second feature extracting filter **22** computes a variation of Respiratory Sinus Arrythmia (RSA) **32.** This calculation can be based notably on interactions between Heart Rate and Respiration Rate.

Further, a third feature extracting filter **23** computes a variation (DRR) of Respiration Rate (RR) **33.**

Further, a fourth feature extracting filter **24** computes a Heart Rate Variation (HRV) **34.**

Second feature extracting filter **22,** can rely partially on intermediate result(s) provided by the third and fourth feature extracting filter **23, 24.**

Regarding the fourth feature extracting filter **24,** the calculation of heart rate can be based notably on green PPG light response, especially the AC component thereof, namely **AC3.** Parametric time-delay autocorrelation allows to determine the actual time delay from one heart cycle to the next one, e.g. peak-to-peak time of the latest sampled heart cycle. Circular buffer comprising several seconds (like typically 10 seconds) allows to trace back the past peak-to-peak time of the latest sampled heart cycles. HRV **34** is inferred from the variation of the peak-to-peak time of the latest sampled heart cycles.

Regarding the third feature extracting filter **23,** the calculation of Respiration rate RR can be based on the one hand on variations of blood oxygen saturation level SPO2 along a respiration cycle, on the other hand on motion signals filtered with a band pass filter (for example a [0,15 Hz - 0,5 Hz]).

Regarding SPO2, here we can use either the red PPG light response, or the infrared PPG light response, or both to compute respiration rate. A low pass filter with a cutoff frequency around 0,5 Hz allows to find the maxima and minima of the low passed signal. Parametric time-delay autocorrelation allows to determine the actual time delay from one respiration cycle to the next one, e.g. peak-to-peak time of the latest sampled respiration cycle. Circular buffer comprising several dozens of seconds (like typically 1 minute) allows to trace back the past peak-to-peak time of the latest sampled respiration cycles. Band passed filtered motion signals are also taken as first order in the third feature extracting filter **23.** Thereafter, Variation of respiration rate DRR **33** is inferred from the variation of the peak-to-peak time of the latest sampled respiration cycles.

Respiration rate variability increases during respiratory events.

Regarding the second feature extracting filter **22,** it uses the same process as described above to identify and locate the maxima and minima of, on the one hand green PPG light response, and on the other hand either the red PPG light response, or the infrared PPG light response, or both. Respective relative positions of the respiration and heart peaks, allows to compute the RSA.

The RSA causes the heart rate to fluctuate with each inspirations and expirations, so during an apnea event, the reduction of the airflow causes the high frequency heart rate variations to lower.

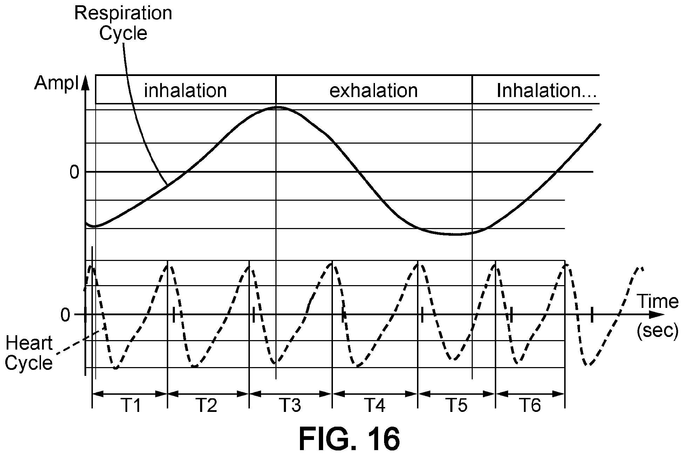

Respiratory Sinus Arrythmia RSA is modulated by the respiration intensity, which lowers during respiratory events. This is shown at Figure 16, where first heart rate cycles durations **T1** and **T2** occur when inhalation takes place, whereas subsequent heart rate cycles duration **T3, T4** and **T5** occur when exhalation takes place. Heart rate cycles at exhalation are longer than heart rate cycles at inhalation in a changing magnitude which is of particular interest in the present disclosure.

RSA can be assessed here either as a ratio of the longest heart rate cycle time divided by the shortest heart rate cycle time, this taken along the latest or the two most recent respiration cycles, or can be assessed as a difference between the longest heart rate cycle time and the shortest heart rate cycle time, this taken along the latest or the two most recent respiration cycles.

In a normal breathing condition, RSA is rather high, whereas RSA decreases when breathing condition deteriorates, whether it is obstructive or a central apnea. Variation of this ratio over several respiration cycles gives the second pattern channel variation of Respiratory Sinus Arrythmia (RSA) **32.**

Further, a fifth feature extracting filter **25** computes a motion index **35.**

Short bursts of movements are sometimes present at the end of respiratory events.

In one embodiment the motion index is calculated as follows.

$MI = \sum \alpha Ax + \beta AY + \gamma AZ + \sum \varphi \omega X + \sigma \omega Y + \theta \omega A$, which gives the motion index pattern channel **35**. $\alpha$, $\beta$, $\gamma$, $\varphi$, $\sigma$, $\theta$ are weight coefficients, which depend on the type of the device and the anatomical site.

Since the motion index combines linear acceleration and rotational speed, the motion index can be named a motion composite index.

In a variant, motion index pattern channel 35 is only based on linear accelerations or only based on rotational speeds.

Further, a sixth feature extracting filter **26** computes a variation (DACPPGi) of AC component of PPG light response. AC component is extracted here as explained above. Although any of the three wavelength responses can be used, we use preferably here the green PPG light response ($\lambda$**3**, LED **13**).

As shown at figure 8, the feature extracting filter extracts the extrema of the AC component, namely the maxima MaxAC(i) and the minima MinAC(i). The peak-to-peak amplitude [MaxAC(i) - MinAC(i)] is of particular interest. The variation of the AC peak-to-peak amplitude is also of particular interest, it can be computed as :

$$([MaxAC(i) - MinAC(i)] - [MaxAC(i-1) - MinAC(i-1)])$$

Since AC of the green PPG response decreases sharply after respiratory events, this sixth pattern channel **26** is used by the downstream computation as will be seen below.

Also AC of the green PPG response is modulated by the respiration intensity, which lowers during respiratory events.

Variations of the PPG AC component is computed to output the sixth pattern channel **36.**

Further, a seventh feature extracting filter **27** computes a variation (DDCPPGi) of DC component of PPG light response. AC component is extracted here as explained above. Although any of the three wavelength responses can be used, we use preferably here the green PPG light response (LED 13).

Variations of the green PPG DC component is computed to output the seventh pattern channel **37.** As shown at figure 8, the green PPG DC component decreases in the shown timeframe.

Since DC of the green PPG response is modulated by the respiration intensity, which lowers during respiratory events, this seventh pattern channel **27** is used by the downstream computation as will be seen below.

Each of the feature extracting filters **21-27** performs its computation at the first sampling frequency **F1.**

The feature extracting filters **21-27** comprises one or more of : extrema detection, normalization, nonlinear rescaling, autocorrelation, cross correlation, exponential filtering, etc. Such type of feature extracting filters are lightweight algorithms.

In one particular embodiment, the plurality of pattern channels comprise at least one or more of the following:

**31-** Variation (DSpO2) of blood oxygen saturation level (SpO2),
**36-** variation (DACPPGi) of AC component of PPG light response,
**37-** variation (DDCPPGi) of DC component of PPG light response.

In this particular embodiment, for the said PPG light response, the PPG light response is the green wavelength PPG light response.

The plurality of pattern channels may additionally comprise the 4 other pattern channels:

**32-** Variation (DRSA) of Respiratory Sinus Arrythmia (RSA),
**33-** Variation (DRR) of Respiration Rate (RR),
**34-** Heart Rate Variation (HRV),
**35-** Motion index.

The pattern channels **31-37,** outputted by the feature extracting filters, may pass through a down-sampling process, i.e. down-sampling sub-sampling from the first sampling frequency **F1** to a second frequency **F2,** said second frequency **F2** being comprised between 0,5 Hz and 2Hz, preferably around 1 Hz.

A circular buffer for each pattern channel contains a predefined window depth. The predefined window depth may be about 1 minute.

Downstream the feature extracting filters **21-27,** the method provides a step denoted **c-** consisting in applying, at the control unit **6,** a computation engine **5** to the plurality of pattern channels **31-37,** preferably after the abovementioned down-sampling.

The output of the computation engine **5** is configured to detect a breathing disturbance event (BDE in short) **38.**

The breathing disturbance event is inferred with an a-posteriori logic, considering rolling windows of 10s, 20s 30 s 1min, or 2min.

Neural Network

The computation engine 5 may be a Neural Network.

Among various possibilities to embody such computation engine, it is promoted herein a one dimension convolutional Neural Network **54,** termed in short 1DCNN.

However, it should be noted that the computation engine can be embodied in a convention full layer neural network, or even in a conventionally programmed rules.

Also the neural network can be a LSTM Neural Network, i.e. a Long Short Term Memory Neural Network. We may use recurrence of LSTM neural network to provide another optimal solution for the computation engine. Other recurrent neural network topologies are not excluded.

Figure 10 illustrates one embodiment of such one dimension convolutional neural network **54.**

The 1D Convolutional Neural Network (1DCNN) comprises an input layer **inL** fed by the seven pattern channels **31-37,** as built above.

More precisely, as shown at figure 10, the seven pattern channels **31-37** are injected into the input layer as values of a circular buffer representing a rolling window of the more recent calculation. The depth/width of the window can be as large as 100 for each channel (e.g. from **i-99** to **i**) namely this means about 700 points/neurons at the input layer.

The number of points can be made dependent on the required time frame, i.e. from 30 seconds to 10 minutes for instance. Each pattern channel may have a different time frame depth. In a preferred embodiment, all the pattern channels have the same time frame depth (consistent sampled data).

Also, the total number of points depends on the sampling frequency of raw data series. According to another option, the depth/width of the window can represent a certain time of sampling, said certain time can be comprised between 30 seconds and 600 seconds, comprised between 60 seconds and 120 seconds.

At each clock tick of the second frequency, a new value is inserted in each pattern channel inputted in the 1DCNN, the rest of the values are shifted by one position to the past.

The number of hidden layers can range from 2 to 10 or more. Some of the hidden layers can be pooling layers.

Not only the first hidden layer can be a 1D convolutional Neural Network, there may be provided an additional 1D convolutional Neural Network in one further hidden layer, i.e. several convolutional layers can be provided in the neural network.

In the example promoted here, the 1D Convolutional Neural Network has for the convolution function a 'width' of 3 to 20 neuron links.

According to one option, the output layer **outL** may comprise a single neuron, the value of which is the outputted BDE rating value.

Alternately, according to another option, the output layer **outL** may comprise a limited number of neurons, one for each apnea intensity. For example, there can be 4 neurons in the output layer:

N1- no apnea
N2- hypopnea / light apnea
N3- apnea event
N4- severe apnea event.

There is provided a deep learning process, configured to tweak the various weights involved in the one dimension convolutional Neural Network 54. The learning process is carried out from a set patients undergoing polysomnography with various additional sensors, in the frame of one or more clinical test(s).

In the illustrated example, the one dimension convolutional Neural Network **54** exhibits less than 5000 weight coefficients, and exhibits less than 2500 floating point multiplications per second.

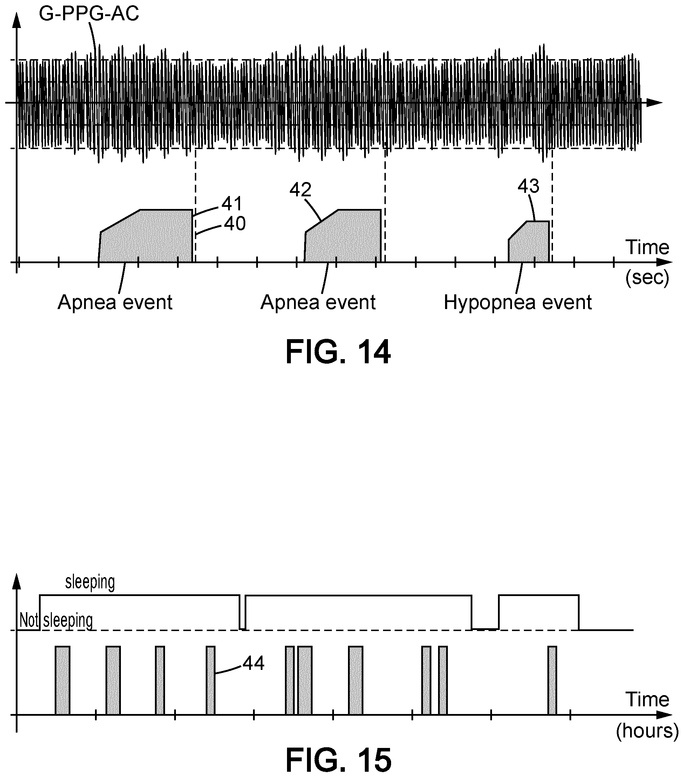

Figure 14 gives an example of time chart. Here the timeframe is in seconds, we consider here a time scale of several seconds to several dozens of seconds Only the green AC response G-PPG-AC is shown among the three PPG responses.

At time **40,** the occurrence and duration of an apnea event **41** is determined by the neural network **54.** In the illustrated example, another shorter apnea event **42** is later determined. In the illustrated example, a hypopnea event **43** is later determined.

The computation also comprises a 'Long term' post processing step **56.** In practice, the method comprises a step of determining an Apnea/Hypopnea Index (AHI).

Figure 15 gives another example of a time chart but at a larger scale. The timeframe is in hours, we consider here a time scale of several hours which represent usually a full night sleep. The chart displayed can resembles a night graphical synthesis that can be displayed at the smartphone **89.** Each apnea event **44** is shown by a bar. The height and/or width of the bar may be representative of a macroscopic importance of the apnea event or sequence of close apnea events. The top line denotes the sleep stat of the user. The bottom line shows apnea events in a timely manner.

As illustrated at Figure 13, such Apnea/Hypopnea Index (AHI) is a weighed calculation of a number of breathing disturbance event **38** over a total/cumulated sleep duration.

For example, the Apnea/Hypopnea Index **91** can give : "7 events per hour" or "13 events per hour". This can be synthetized by light apnea rating, or medium light apnea rating, or even severe light apnea rating.

This supposes to compute the sleep duration. This is obtained from a sleep status input **94.**

A synthetic report of the past night can therefore be issued for the user and/of for its caretaker. The synthetic report of the past night is considered a score of sleep quality and/or reveal light or severe apnea syndrome.

In addition, the 'Long term' post processing step **56** can take into account user data history.

Wristwatch example

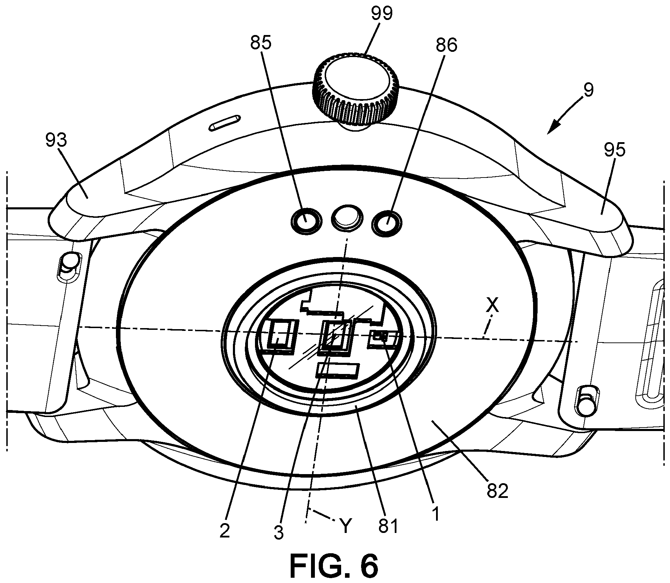

The wristband **19** is removably attached to the pairs of arms **93, 95** via two shafts **17,18** (cf Figures 1 and 6). The abovementioned shaft allows a pivot connection of the wrist band with respect to the main casing **90.**

The control unit **6** is housed in an upper area within the wristwatch. The control unit **6** is configured to handle the time display.

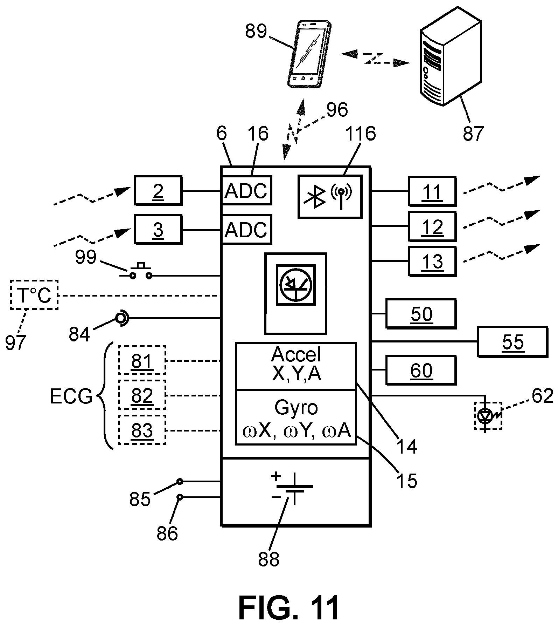

As shown at figure 11, the wristwatch comprises a battery **88.** The battery **88** is preferably a rechargeable type battery, for which there are provided charging pins **85 86.**

As known for a wristwatch, there is provided current time display at the top/front face of the watch. Here, there are provided physical hands **55,** each formed as a stick pivotally mounted around axis A. However hands different from physical stick(s) can also be considered in the present scope. A completely digital solution can also be envisioned.

There is provided a control knob **99.** This knob can be rotated around its axis and pushed along its axis. There is provided a front bezel ring **83,** distinct from the main casing **90,** the purpose of which will be seen later.

Visible from the front side of the watch, there are provided two pixelated digitally controlled area **50, 60.** On said digitally controlled areas **50, 60,** one displayed function is a pedometer function, other displayed data relate to or more bio-parameter(s). Some displayed functions are permanent, other are temporarily displayed.

Last night apnea score) can be displayed in one of the digitally controlled area **50, 60.** Also lastly measured SpO2 (arterial blood oxygen saturation of hemoglobin) can be displayed. A result of an ECG measurement can also be temporarily displayed.

There are provided three ECG electrodes : the ring member **81,** the annular cup member **82** and the front bezel ring **83.**

Regarding geometric arrangement, the emission point of infrared LED 11 is at a first location denoted **P1,** i.e. the infrared LED **11** is arranged to emit light rays at the first position **P1.** The emission point of red LED 12 is at a second location denoted **P2,** i.e. the red LED **12** is arranged to emit light rays at the second position denoted **P2.** The emission point of green LED 13 is at a third location denoted **P3,** i.e. the green LED **13** is arranged to emit light rays at the third position denoted **P3.**

The broadband photodiode **2** is generally at a fourth location having a center position denoted **P4.** The selective photodiode **3** is generally at a fourth location having a center position denoted **P5.**

We note e4 = Dist **(P1,P4)** = Dist **(P2,P4).** In the illustrated example (cf Fig 3 and 4), **e4** is at least 7 mm
It should be noticed that the light path for red light rays is nearly identical to the light path for infrared light rays. Indeed P1 and P2 are spaced by a distance less that 1 mm, and red / infrared light paths go to the same target namely the broadband photodiode **2.**

We note e5 = Dist **(P3,P5).** In the illustrated example, **e5** is at least 2 mm.

System aspects and miscellaneous

As apparent from figure 11, the wristwatch comprises a wireless coupler **116,** likewise a Bluetooth™ coupler or a Wi-Fi coupler. The wireless coupler **116** enables exchange of data with a smartphone **89.** Communication to a server **87,** through a router is also envisioned.

Results of apnea events monitoring can be transmitted to smartphone **89** and can also be forwarded to a server **87.**

At the system level, user can have an application on his/her smartphone **89.** Thanks to this application, the user can ask for monitoring his/her sleep for the upcoming night. Under this condition, apnea events monitoring only takes place when the user request it.

Therefore, the apnea monitoring only occurs if the user has asked for it, and for all the other nights for which the user has made no request, the device does not perform the apnea monitoring, thereby saving electrical energy. Indeed, sampling the PPG response on a long term like several hours along the night sleep demands a substantial amount of electrical energy in the context of a battery-supplied device.

Although not necessary in the present disclosure, there may be provided a user profile (age, gender, weight, BMI) which can be taken into account in the Apnea/Hypopnea Index (AHI) calculation.

Advantageously, the promoted method is carried out regardless of any user profile. It has been found that the promoted method works well irrespective of a user profile (gender, weight, height, BMI, etc).

Advantageously, the promoted method can further use history data of the user, as a parametrization. Last night mean average HR can be taken as a reference at least for the beginning of the sleep sequence.

According to one option, the device (wristwatch or other) comprises a worn test function. The worn condition can be determined by comparing the light response with a range of possible light response expected when the device is applied to a human tissue.

According to one option, the worn test function relies on the infrared LED. Namely, when the worn is assumed to be false, a new worn test is carried out periodically, for example every 10 seconds or 30 seconds, by activating the infrared LED and checking the IR light response. The period of checking can be made dependent on the motion signals delivered by the motion sensor. For example, if the motion signals are null or below a small threshold, the worn checking test can be slowed down to one every 10 minutes. And conversely, when motion signals are greater, the worn checking test can be made on a much faster pace.

When the worn test function gives a negative result, the data acquired from the sensors are disregarded. Downstream computation can be halted. Further, electrical energy can be spared whenever the device is not worn.

As shown at figures 2 and 11, according to one option, the method can include displaying the so determined current blood oxygen saturation level on a display intended to be looked at by a user. According to one option, the method can include providing the so determined current blood oxygen saturation level to a remote entity, preferably via a wireless transmission. Results are therefore displayed directly to the user/wearer and can be forwarded to another device like a smartphone **89** either belonging to the user or to some medical staff/caretaker. Results can also be forwarded to a server **87.**

In a wristwatch example, regarding the rechargeable battery **88** and its autonomy, with a battery nominal capacity comprised between 150 mAh and 300 mAh, although the wristwatch is configured to continuously monitor the sleep respiration, its electrical energy efficiency is outstanding, allowing several days of autonomy before needing to recharge.

In many embodiments of such devices, we note that the promoted method is performed within a device formed as an integral device, i.e. a single unit. Such unit houses herein said control unit **6,** LEDs, light and motion sensors, and possibly a battery. It can be autonomous for assessing a breathing disturbance event, without requiring external further sensor.

**Claims**

1. A method to detect breathing disturbances of a user, carried out in a single/integral device comprising a plurality of LEDs (11,12,13) configured to emit light rays at least three wavelengths ($\lambda1, \lambda2, \lambda3$), at least a light sensing device (2,3), a control unit (6) and at least a motion sensor (14,15), the method comprising :

   a- acquiring, at a first sampling frequency (F1), a photoplethysmography (PPG) light response for each wavelength of the plurality of at least three wavelengths, resulting respectively in a first, second and third raw data series representing an amplitude response through a user's tissue, and acquiring motion signals from the motion sensor, resulting in a fourth raw data series,

   b- applying, at the control unit (6), a plurality of feature extracting filters (21-27) to the first, second, third and fourth raw data series, each of the plurality of feature extracting filters outputting one of a plurality of corresponding pattern channels (31-37), the plurality of pattern channels comprising at least one or more of:

   - variation (DSpO2) of blood oxygen saturation level (SpO2),
   - variation (DRSA) of Respiratory Sinus Arrythmia (RSA),
   - variation (DRR) of Respiration Rate (RR),
   - Heart Rate Variation (HRV),
   - Motion index,
   - variation (DACPPGi) of AC component of PPG light response,
   - variation (DDCPPGi) of DC component of PPG light response

   c- applying, at the control unit (6), a computation engine (5) to the plurality of pattern channels, and detecting therefrom a breathing disturbance event (BDE), wherein the computation engine (5) comprises a neural network.

2. The method according to claim 1, wherein the neural network of the computation engine (5) comprises a one dimension convolutional Neural Network (1DCNN).

3. The method according to claim 1 or 2, further comprising a step of determining an Apnea/Hypopnea Index (AHI) computed from a calculation of a number of breathing disturbance event (BDE) over a total/cumulated sleep duration.

4. The method according to any one of claims 1 to 3, wherein the plurality of pattern channels comprises at least:

- variation (DSpO2) of blood oxygen saturation level (SpO2),
- variation (DACPPGi) of AC component of PPG light response, said PPG light response being the green wavelength PPG light response,
- variation (DDCPPGi) of DC component of PPG light response, said PPG light response being the green wavelength PPG light response,

and the plurality of pattern channels comprises at least one or more of:

- variation (DRSA) of Respiratory Sinus Arrythmia (RSA),
- variation (DRR) of Respiration Rate (RR),
- Heart Rate Variation (HRV),
- Motion index.

5. The method according to claim 2 and 3, wherein the one dimension convolutional Neural Network (1DCNN) comprises an input layer fed by latest values of the plurality of pattern channels.

6. The method according to claim 3, further comprising one or more of:

- displaying the Apnea/Hypopnea Index (AHI) on a display intended to be looked at by a user
- providing Apnea/Hypopnea Index (AHI) to a remote entity (87,89), preferably via a wireless transmission.

7. The method according to any one of claims 1 to 6, wherein the pattern channels, outputted by the feature extracting filters, comprises a down-sampling from the first sampling frequency (F1) to a second frequency (F2), said second frequency (F2) being comprised between 0,5 Hz and 2Hz, preferably around 1 Hz.

8. The method according to any one of claims 1 to 7, wherein the plurality of at least three wavelengths ($\lambda 1$, $\lambda 2$, $\lambda 3$) comprises :

- a first wavelength ($\lambda 1$) which has a center emission wavelength comprised between at 920 nm and 960 nm and forms an infrared LED,
- a second wavelength ($\lambda 2$) which has a center emission wavelength comprised between at 650 nm and 665 nm and forms a red LED,
- a third wavelength ($\lambda 3$) which has a center emission wavelength comprised between 480 nm and 540 nm and forms a green or blue LED.

9. The method according to any one of claims 1 to 8, wherein the light response for each wavelength are taken at a user's wrist.

10. The method according to claim 9, wherein steps **a-** to **c-** are implemented in a control unit housed in a wrist-worn device, preferably a wristwatch.

11. The method according to any one of claims 1 to 10, further comprising a worn test function, wherein whenever the worn test function gives a negative result, at least displaying and outputting of the user breathing disturbance event and/or Apnea/Hypopnea Index (AHI) are suspended, and possibly the computation is also suspended.

12. The method according to any one of claims 1 to 11, wherein the predefined first sampling frequency (SF) is comprised between 15 Hz and 40Hz, preferably comprised between 15 Hz and 40Hz, more preferably comprised between 20 Hz and 30Hz.

13. The method according to any one of claims 1 to 12, wherein at the step a-, the acquisition of PPG photoplethysmography light response is performed by two photodiodes, i.e. a broadband photodiode (2) configured to receive red and infrared light rays, and a selective photodiode (3) configured to receive green light rays.

14. The method according to any one of claims 1 to 13, at least step **b-** is performed each time a new set of wavelength light responses is acquired and motion signals are acquired, namely the feature extracting filters (21-27) are timely triggered according to the first sampling frequency clock.

15. A device comprising a plurality of LEDs (11,12,13) configured to emit light rays at least three wavelengths ($\lambda 1$, $\lambda 2$, $\lambda 3$), at least a light sensing device (/photodiode)(2,3), a control unit (6), and at least a motion sensor (14,15), the device being configured to carry out a method according to any one of claims 1 to 14.

**FIG. 1**

**FIG. 2**

EP 3 906 853 A1

FIG. 3

FIG. 4

16

**FIG. 5**

FIG. 6

FIG. 7

FIG. 8

51

Raw
IR

DC1

AC1

57      52

Raw
R

DC2

AC2

53      58

Raw
G

DC3

AC3

59

# FIG. 9

**FIG. 10**

FIG. 11

FIG. 12

FIG. 13

**FIG. 14**

**FIG. 15**

FIG. 16

Europäisches
Patentamt

European
Patent Office

Office européen
des brevets

# EUROPEAN SEARCH REPORT

Application Number

EP 20 30 5439

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D | US 2019/282159 A1 (GOWDA SURAJ [US] ET AL) 19 September 2019 (2019-09-19) | 1,3-6, 8-11, 13-15 | INV. A61B5/1455 A61B5/11 |
| Y | * paragraph [0006] - paragraph [0007] * <br> * paragraph [0024] - paragraph [0025] * <br> * paragraph [0040] - paragraph [0044] * <br> * paragraph [0054] * <br> * paragraph [0057] - paragraph [0062] * <br> * paragraph [0071] * <br> * paragraph [0079] * <br> * paragraph [0090] * <br> * figures 1A,1B,5,6 * | 2,7,12 | A61B5/00 |
| Y | US 2018/015282 A1 (WANER MILTON [US] ET AL) 18 January 2018 (2018-01-18) <br> * paragraph [0119] * | 2 | |
| Y | CN 111 035 395 A (SHANGHAI 6TH PEOPLES HOSPITAL; UNIV SHANGHAI JIAOTONG) 21 April 2020 (2020-04-21) <br> * paragraph [0081] * | 7 | |
| Y | US 2017/281050 A1 (NOGUCHI YOSHIHIRO [JP] ET AL) 5 October 2017 (2017-10-05) <br> * paragraph [0112] * | 12 | TECHNICAL FIELDS SEARCHED (IPC) <br><br> A61B |
| A | US 2015/164411 A1 (SELVARAJ NANDAKUMAR [US] ET AL) 18 June 2015 (2015-06-18) <br> * paragraph [0016] * <br> * paragraph [0018] * <br> * paragraph [0022] * <br> * paragraph [0026] * <br> * paragraph [0032] - paragraph [0033] * | 1-15 | |

The present search report has been drawn up for all claims

| Place of search | Date of completion of the search | Examiner |
|---|---|---|
| Berlin | 1 October 2020 | Völlinger, Martin |

**EP 3 906 853 A1**

## ANNEX TO THE EUROPEAN SEARCH REPORT
## ON EUROPEAN PATENT APPLICATION NO.

EP 20 30 5439

This annex lists the patent family members relating to the patent documents cited in the above-mentioned European search report.
The members are as contained in the European Patent Office EDP file on
The European Patent Office is in no way liable for these particulars which are merely given for the purpose of information.

01-10-2020

| Patent document cited in search report | | Publication date | Patent family member(s) | | Publication date |
|---|---|---|---|---|---|
| US 2019282159 | A1 | 19-09-2019 | CN | 111246798 A | 05-06-2020 |
| | | | EP | 3668396 A1 | 24-06-2020 |
| | | | US | 2019053754 A1 | 21-02-2019 |
| | | | US | 2019282159 A1 | 19-09-2019 |
| | | | WO | 2019036661 A1 | 21-02-2019 |
| US 2018015282 | A1 | 18-01-2018 | US | 2018015282 A1 | 18-01-2018 |
| | | | WO | 2018017508 A1 | 25-01-2018 |
| CN 111035395 | A | 21-04-2020 | NONE | | |
| US 2017281050 | A1 | 05-10-2017 | CN | 107106080 A | 29-08-2017 |
| | | | DE | 112015005804 T5 | 19-10-2017 |
| | | | JP | 6371410 B2 | 08-08-2018 |
| | | | JP | WO2016104538 A1 | 02-11-2017 |
| | | | US | 2017281050 A1 | 05-10-2017 |
| | | | WO | 2016104538 A1 | 30-06-2016 |
| US 2015164411 | A1 | 18-06-2015 | US | 2015164410 A1 | 18-06-2015 |
| | | | US | 2015164411 A1 | 18-06-2015 |
| | | | WO | 2015089382 A1 | 18-06-2015 |
| | | | WO | 2015089387 A1 | 18-06-2015 |

EPO FORM P0459

For more details about this annex : see Official Journal of the European Patent Office, No. 12/82

**EP 3 906 853 A1**

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2019282159 A
- US 2015289802 A